# EUROPEAN PATENT APPLICATION

(11) **EP 3 065 073 A2**
(43) Date of publication of application: **07.09.2016**
(21) Application number: 16158096.4
(22) Date of filing: 01.03.2016
(51) Int. Cl.: G06F 19/00

(54) **AVAILABILITY SCHEDULING FOR PATIENT SUPPORTS**

(30) Priority: 02.03.2015 US 201562126851 P
(71) Applicant: Hill-Rom Services, Inc., Batesville, IN 47006-9167 (US)
(72) Inventor: IUCHA, Florin, Cary, NC North Carolina 27518 (US); MOYLAN, Thomas H, Hanson, MA Massachusetts 02341 (US)
(74) Representative: Findlay, Alice Rosemary

(57) **Abstract**

A patient support apparatus includes a frame comprised of one or more components controlled by a controller to move portions of the frame between a plurality of positions and orientations. The controller monitors usage of the patient support apparatus components and dates when maintenance has been performed on one or more of the patient support apparatus components. The controller is operable to transmit the maintenance date and usage information to a remote computing device, wherein the remote computing device is configured to determine an availability status of the patient support apparatus that indicates whether the patient support apparatus is available to be scheduled for maintenance or assigned to a patient, based on the maintenance date and the usage information.

## Description

The present disclosure relates to patient supports and in particular, to transmitting data communications from patient supports to a remote computing device. More particularly, the present disclosure relates to transmitting usage data of patient supports to a remote computing device for determining a scheduling availability of the patient supports.

Modern patient supports, such as hospital beds and lifts, typically have various electronically controlled mechanisms, such as height adjustment mechanisms and actuators, which allow various portions of the patient supports to assume different configurations at varying distances from the floor. The electronically controlled mechanisms, actuators, and various other electronic components are generally controlled by a central controller, which interprets inputs from various input interfaces of the patient support and provides signals to the electronically controlled mechanisms to make particular adjustments in response to the inputs. Such electronically controlled mechanisms may require maintenance (i.e., regularly scheduled, preventative maintenance) to be performed periodically to keep them functioning properly, or to be maintained within certain regulations. In addition to the height control mechanisms, certain patient supports, such as hospital beds, for example, typically include a mattress. In such patient supports, the mattress may be an inflatable mattress which may require additional hardware, such as an air pump and various valves that may also be controlled by the controller. As such, not only does the inflatable mattress typically need changed periodically, but similar to the height adjustment mechanisms and actuators, the mattress inflation hardware may also require periodic maintenance to be performed.

An apparatus according to the present disclosure includes one or more of the features recited below alone, or in any combination.

In a first aspect of the present disclosure, a patient support apparatus comprises a frame that includes a base frame coupled to an intermediate frame configured to move vertically relative to the base frame, and a deck coupled to the intermediate frame configured to move between a plurality of positions relative to the intermediate frame. The patient support apparatus further comprises a controller in electrical communication with one or more components to control movement of the intermediate frame and the deck.

In some embodiments, the one or more components of the patient support apparatus include at least one of a valve, an actuator, and a sensor.

In some embodiments, the apparatus includes a data storage device to store a usage data of the patient support apparatus, and the communication circuitry operable to transmit the usage data to a remote computing device via a network to determine the availability status of the patient support apparatus based on the usage data.

In some embodiments, the controller includes an interface to receive an indication that maintenance was performed on the patient support apparatus. The controller further includes a data storage device to store a maintenance date that corresponds to a date and time the indication was received. The communication circuitry is further operable to transmit the maintenance date via the network to the remote computing device to determine an availability status of the patient support apparatus to indicate whether the patient support apparatus is available to be scheduled for maintenance or assigned to a patient, wherein the availability status is based on the maintenance date.

In some embodiments, the patient support apparatus further comprises an inflatable mattress supported by the deck, a pump to inflate the inflatable mattress, and a pressure actuator valve to control a pressure of the inflatable mattress, the pressure actuator valve being fluidly coupled between the inflatable mattress and the pump. The controller is in electrical communication with the pressure actuator valve to provide a signal to cause the pressure actuator valve to adjust the pressure of the inflatable mattress. The usage data includes a pressure actuator valve usage based at least in part on a number of times the pressure actuator valve was used, which may be since the maintenance date.

In some embodiments, the patient support apparatus further comprises a lift mechanism that includes one or more lift arms that interconnect the base frame and the intermediate frame to cause the intermediate frame to move relative to the base frame. The controller is in electrical communication with the one or more lift arms to provide signals to the one or more lift arms to control the movement of the intermediate frame relative to the base frame. The usage data includes an actuator usage of the lift mechanism.

In some embodiments, the actuator usage of the lift mechanism includes at least one of a number of hi/low cycles that indicate a number of times the lift mechanism moved the intermediate frame from hi to low positions and/or an amount of time the lift mechanism has spent in operation and/or a number of activations of the lift mechanism, all or which may be since the maintenance date.

In some embodiments, the patient support apparatus further comprises one or more actuators to control the movement of the deck between a plurality of positions relative to the intermediate frame. The controller is in electrical communication with the one or more actuators to provide signals to the one or more actuators to control the movement of the deck between the plurality of positions. The usage data includes an actuator usage amount that indicate a number of times the one or more actuators have been used to move the deck, which may be since the maintenance date.

In some embodiments, the communication circuitry is further operable to transmit the usage data to the remote computing device via the network to determine the availability status of the patient support apparatus based on the maintenance date and the usage data.

In some embodiments, the remote computing device is further to provide an availability status that indicates the patient support apparatus is available to be assigned to a patient in response to a determination that the patient support apparatus is presently assigned to a patient or the usage data indicates a usage threshold was not exceeded.

In some embodiments, the controller further includes a real-time clock, and wherein the controller is further to determine whether the patient support apparatus is due for maintenance and to transmit an indication to the remote computing device that indicates the patient support apparatus should be scheduled for maintenance in response to a determination that the patient support apparatus is due for maintenance.

In some embodiments, the determination that the patient support apparatus is due for maintenance comprises a determination that a predetermined amount of time has elapsed since the maintenance date based on a present value of the real-time clock.

According to another aspect of the present disclosure, a system for managing availability of a patient support for scheduling the patient support comprises a patient support that includes a frame including one or more components controlled by a controller to move portions of the frame between a plurality of positions. The system further comprises a remote computing device to manage whether the patient support is to be set as scheduled for maintenance or set available for assignment to a patient. The controller includes an interface to receive an indication that maintenance was performed on the patient support apparatus, a data storage device to store a maintenance date that corresponds to a date and time the indication was received, and communication circuitry operable to communicate with the remote computing device to transmit the maintenance date to the remote computing device. The remote computing device is to determine whether the patient support apparatus is available to be scheduled for maintenance or assigned to a patient based on the maintenance date.

In some embodiments, the remote computing device is further to provide an availability status that indicates to schedule the patient support apparatus for maintenance in response to a determination that the patient support apparatus is not presently assigned to a patient and is due for maintenance.

In some embodiments, the remote computing device is further to provide an availability status that indicates the patient support apparatus is available to be assigned to a patient in response to a determination that the patient support apparatus is presently assigned to a patient or is not due for maintenance.

In some embodiments, the controller further includes a real-time clock and the controller is further configured to determine whether the patient support is due for maintenance based on a comparison of a present time determined from the real-time clock and the maintenance date, and wherein the patient support is further to transmit an indication to the remote computing device that indicates the patient support apparatus should be scheduled for maintenance in response to a determination that an amount of time has passed since the maintenance date that exceeds a predetermined duration.

In some embodiments, the data storage device of the controller is further configured to store usage information that corresponds to a usage of the one or more components of the frame, and wherein the patient support is further to transmit the usage information to the remote computing device.

In some embodiments, the remote computing device is further to provide an availability status that indicates to schedule the patient support apparatus for maintenance in response to a determination that the patient support apparatus is not presently assigned to a patient and the usage information indicates a usage threshold was exceeded.

The invention will now be further described by way of example with reference to the accompanying drawings, in which:
Fig. 1 is a block diagram of a system for managing the availability scheduling of a patient support for maintenance according to one embodiment of the present disclosure;
Fig. 2 is side view of an embodiment of the patient support of Fig. 1;
Fig. 3 is a block diagram of an embodiment of a controller of the patient support of Fig. 1;
Fig. 4 is a flow diagram of a process for resetting maintenance and usage information that may be executed by the controller of Fig. 3;
Fig. 5 is a flow diagram of a process for determining whether the patient support of Fig. 1 is due for maintenance that may be executed by the controller of Fig. 3;
Fig. 6 is a flow diagram of a process for determining whether the patient support of Fig. 1 is due for maintenance that may be executed by a patient support management system of Fig. 1; and
Fig. 7 is a flow diagram of a process for determining the scheduling availability of the patient support of Fig. 1 that may be executed by a patient support scheduling system of Fig. 1.

In the following description, numerous specific details such as logic implementations, types and interrelationships of system components, and logic partitioning/integration choices are set forth in order to provide a more thorough understanding. In other instances, control structures, gate level circuits and full instruction sequences have not been shown in detail in order not to obscure the invention.

Embodiments of the invention may be implemented in hardware, firmware, software, or any combination thereof. Embodiments of the invention may also be implemented as instructions stored on a machine-readable medium, which may be read and executed by one or more processors. A machine-readable medium may include any mechanism for storing or transmitting information in a form readable by a machine (e.g., a computing device). For example, a machine-readable medium may include read only memory (ROM); random access memory (RAM); magnetic disk storage media; optical storage media; flash memory devices; and others.

The following description describes techniques for managing whether a patient support is to be made available for assignment to a patient based upon usage of the patient support including whether the patient support is due for maintenance (i.e., regularly scheduled, preventative maintenance). The availability scheduling system may assign a patient support to be available to be assigned to a patient based on a determination that the patient support is not due for maintenance. Alternatively, the availability scheduling system may assign a patient support to be scheduled for maintenance and thus not available to be assigned to a patient based on a determination that the patient support is not presently assigned to a patient and due for maintenance. The determination of whether the patient support is due for maintenance may be based on an amount of time that has elapsed since a most recent date (i.e., date and time) that maintenance was performed on the patient support.

In some embodiments, the determination may be additionally or alternatively based on a usage of one or more components of the patient support which may be from the most recent date that maintenance was performed on the patient support. For example, an amount of time that a height adjustment mechanism has been used to adjust the height of a patient support, such as a hospital bed, may be aggregated from the date of the most recent date that maintenance was performed on the patient support. The amount of time the height adjustment mechanism has been used to adjust the height of the patient support may be compared against a predetermined threshold to determine when to trigger an indication that the patient support is again due for maintenance. Upon the indication being triggered, the availability scheduling system may schedule the patient support for maintenance and notify a user of the availability scheduling system that the patient support is not available to be assigned to a patient.

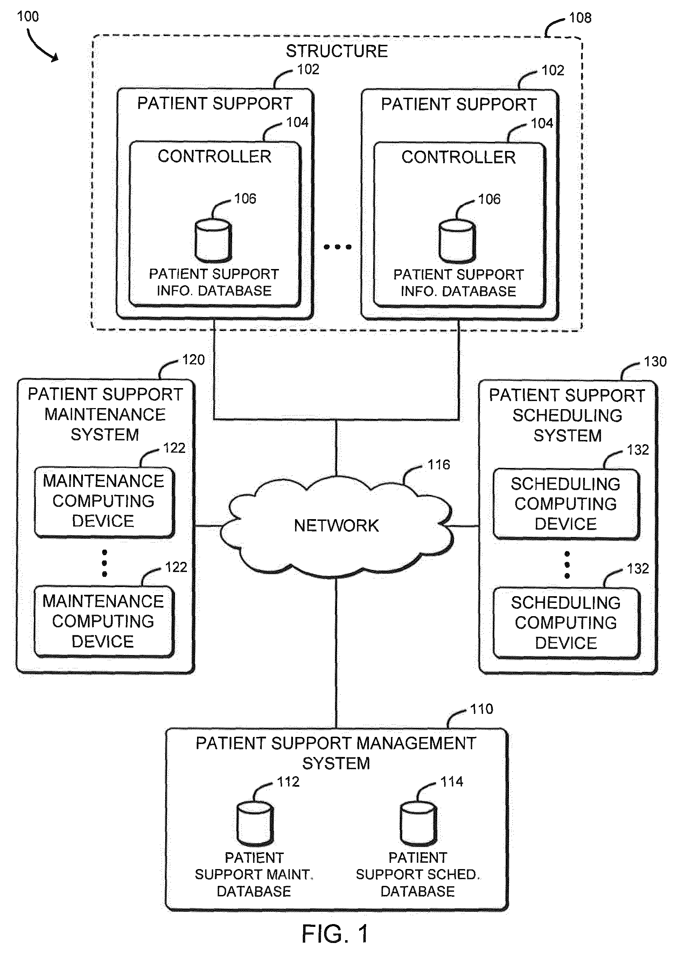

Fig. 1 shows a system 100 in accordance with the present disclosure for determining a scheduling availability for one or more patient supports 102, such as patient beds and/or lifts, for example. Each of the patient supports 102 may transmit information (i.e., usage data) to a remote computing device for determining whether a particular patient support 102 is due for maintenance, or whether the patient supports 102 are available to be scheduled to a patient (i.e., not due or scheduled for maintenance). It should be appreciated that, in some embodiments, various usage data corresponding to a number of functions of various components of the patient supports 102 may be transmitted to the remote computing device. It should be further appreciated that maintenance operations (e.g., minor adjustments, major adjustments, etc.) may be triggered based on the usage data.

In the illustrative system 100, each of the patient supports 102 includes a controller 104 for controlling operation of various bed functions, such as height and pressure adjustments, in response to signals from various interface units and/or components of the patient supports 102 as discussed in further detail below. The illustrative controller 104 includes a patient support information database 106, which may be used to store, amongst other information, a maintenance date that corresponds to the most recent date maintenance was performed on at least a portion of the patient support 102 and/or usage data related to the usage of the electrical and/or mechanical components of the patient support 102.

As shown in the illustrative system 100, the patient supports 102 are located at a structure 108, which may be a room, a medical unit of a hospital, a hospital, or any type of facility that uses patient supports 102, for example. While the illustrative system 100 shows the patient supports 102 being located in a single structure 108, it should be appreciated that a number of patient supports 102 may be located in one or more different structures 108. Additionally, it should be further appreciated that the different structures 108 may or may not be located in structures 108 that are adjacently located (e.g., neighboring rooms of the same medical unit). For example, in one embodiment, one or more patient structures 102 may be located in one hospital, while one or more other patient structures 102 may be located in a different hospital.

The illustrative system 100 additionally includes a patient support management system 110, a patient support maintenance system 120, and a patient support scheduling system 130; each of which are in communication with the patient supports 102 via a network 116. In some embodiments, each of the patient support maintenance system 120 and the patient support scheduling system 130 may be in communication via the network 116 with the patient support management system 110, while the patient support management system 110 may be in communication via the network 116 with the patient supports 102. In other words, in some embodiments, the patient support maintenance system 120 and/or the patient support scheduling system 130 may not be in direct communication with the patient supports 102. It should be appreciated that, in some embodiments, one or more of the patient support management system 110, the patient support maintenance system 120, and the patient support scheduling system 130 may be combined into one or more additional or alternative systems supporting the functionality described herein. It should be further appreciated that, in some embodiments, any of the patient support management system 110, the patient support maintenance system 120, and the patient support scheduling system 130 may be located in the same structure 108 as one or more of the patient supports.

The patient support management system 110 may include any number of computing devices including storage servers, compute servers, etc. to communicate with the patient supports 102, the patient support maintenance system 120, and the patient support scheduling system 130. As will be described in further detail, the patient support management system 110 is configured to store data from the patient supports 102 and provide an indication to the patient support maintenance system 120 and/or the patient support scheduling system 130 to indicate whether the patient supports 102 are available to be assigned to patients or should be scheduled for maintenance.

The illustrative patient support system 110 includes a patient support maintenance database 112 and a patient support scheduling database 114. The patient support maintenance database 112 may include component usage and/or maintenance information for each of the patient supports 102 of the system 100. In some embodiments, each patient support 102 may update the most recent maintenance date upon completion of the maintenance having been performed on the patient support 102. Additionally or alternatively, in some embodiments, the patient support maintenance database 112 may include a maintenance date that corresponds to the most recent instance of maintenance having been performed on each of the patient supports 102, or on particular components thereof (e.g., an actuator, a pump, a mattress, etc.). The patient support maintenance database 112 may additionally include usage information (i.e., usage data) for each of the patient supports 102, which may be used to further determine when maintenance is to be scheduled for each of the patient supports 102.

Based on this information, the patient support system 110, or a user with access to the patient support system 110, may determine when maintenance is to be scheduled for each of the patient supports 102. It should be appreciated that the patient support system 110 may include additional (e.g., a patient support location database) or fewer databases, and that the patient support maintenance database 112 and the patient support scheduling database 114 may be combined into a single database in some embodiments.

The patient support scheduling database 114 may include patient and/or scheduling information for each of the patients and/or patient supports 102 of the system 100. For example, the patient support scheduling database 114 may include whether each patient support 102 is presently assigned to a patient, and if so, may additionally include an expected duration that each presently assigned patient support 102 is expected to be assigned to the patient. Based on this information, the patient support system 110, or a user with access to the patient support system 110, may schedule patients to be assigned to certain patient supports 102 that are not presently due and/or scheduled for maintenance.

The network 116 may be configured as any type of wired or wireless communication network, including cellular networks (e.g., Global System for Mobile Communications (GSM)), digital subscriber line (DSL) networks, cable networks, telephony networks, local or wide area networks, global networks (e.g., the Internet), or any combination thereof. Additionally, the network 116 may include any number of additional network communication devices (e.g., routers, switches, hubs, etc.) as needed to facilitate communications between the computing devices of the system 100.

The patient support maintenance system 120 is configured to manage the scheduling of patient supports 102 for maintenance and facilitate the reporting of whether the maintenance was performed, or not. To do so, the illustrative patient support maintenance system 120 includes one or more maintenance computing devices 122. The maintenance computing devices 122 may be embodied as any type of computation or computing device capable of performing the functions described herein. For example, the maintenance computing devices 122 may be embodied as, without limitation, a computer, a smartphone, a tablet computer, a laptop computer, a notebook computer, a mobile computing device, a wearable computing device, a multiprocessor system, a server (e.g., stand-alone, rack-mounted, blade, etc.), a network appliance (e.g., physical or virtual), a web appliance, a distributed computing system, a processor-based system, and/or any type of compute and/or store device. In use, as described in further detail below, the maintenance computing devices 122 are configured to communicate with the patient support management system 110 and allow users of the maintenance computing devices 122 to access at least a portion of the patient support maintenance database 112 of the patient support management system 110 to be used in scheduling the patient supports 102 for maintenance. The maintenance computing devices 122 may be further configured to receive and process maintenance information, such as whether maintenance was performed on a patient support 102 scheduled for maintenance.

The patient support scheduling system 130 is configured to manage the scheduling (i.e., assignment) of patient supports 102 to patients. To do so, the patient support scheduling system 130 includes one or more scheduling computing devices 132. In some embodiments, the patient support scheduling system 130 may be embodied as an admissions, discharge, and transfer (ADT) system, which may additionally include various servers and storage devices to manage patient support 102 requests for patients as the patients are admitted and discharged.

The scheduling computing devices 132 may be embodied as any type of computation or computing device capable of performing the functions described herein, including, without limitation, a computer, a smartphone, a tablet computer, a laptop computer, a notebook computer, a mobile computing device, a wearable computing device, a multiprocessor system, a server (e.g., stand-alone, rack-mounted, blade, etc.), a network appliance (e.g., physical or virtual), a web appliance, a distributed computing system, a processor-based system, and/or any type of compute and/or store device. For example, in an embodiment wherein the patient support scheduling system 130 is embodied as an ADT system, the patient support scheduling system 130 may include an ADT server, an ADT data storage device, and one or more ADT clients. In use, as described in further detail below, the scheduling computing devices 132 are configured to communicate with the patient support management system 110 and allow a user of the scheduling computing devices 132 to access and/or manipulate at least a portion of the patient support scheduling database 114 of the patient support management system 110 to be used in assigning patients to the patient supports 102.

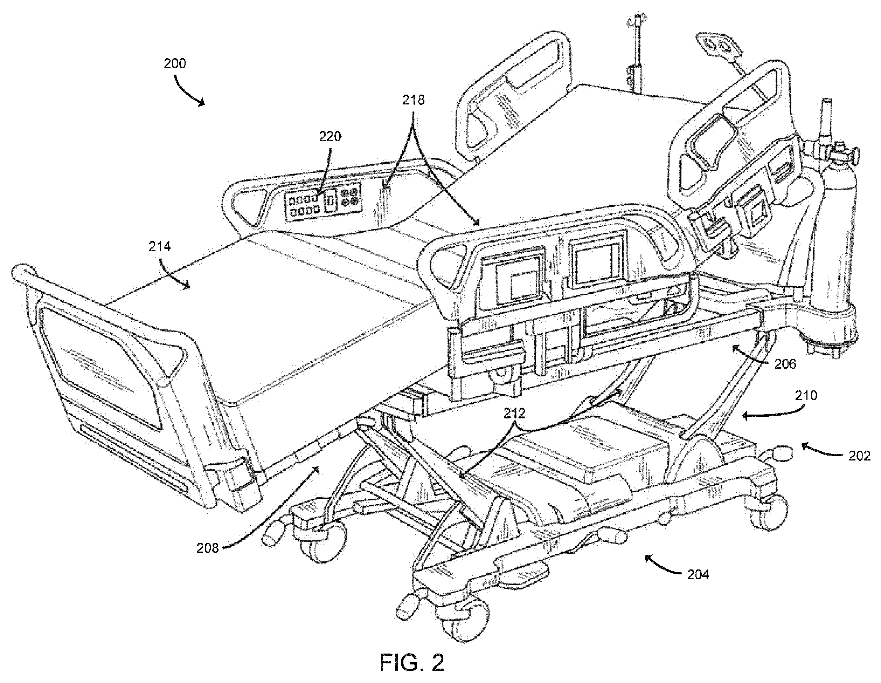

Referring now to Fig. 2, there is illustrated a patient bed 200 that includes a frame 202 in accordance with the present disclosure. The frame 202 includes a base frame 204 that supports a lift mechanism 210 that is comprised of lift arms 212 which extend between the base frame and an intermediate frame 206. The frame 202 additionally includes a deck 208, which is supported on the intermediate frame 206. The illustrative patient bed 200 includes a patient support surface 214 (i.e., a mattress) that may incorporate various functional components, such as inflatable bladders (not shown) whose pressure may be adjusted via a pneumatic pump and various valves (not shown) to regulate the pressure of the inflatable bladders. The patient support surface 214 is positioned on a deck 208, which is supported on an intermediate frame 206.

The lift arms 212 can be raised or lowered to adjust the patient support surface 214 relative to the floor on which the patient bed 200 is located. In some embodiments, the lifts arms 212 may be driven by height adjustment linear actuators (not shown) mounted to the intermediate frame 206. In such embodiments, an upper end of each of the lift arms 212 may be pivotally connected to the intermediate frame 206. In some embodiments, the linear actuators may be coupled to the upper ends of the lift arms 212 by extension links so that extension or retraction of the linear actuators rotates the upper ends of the lift arms 212. Additionally or alternatively, in some embodiments, the linear actuators may be operated independently so that the intermediate frame 206 can be raised, lowered, and/or tilted. The deck 208 may be coupled to one or more deck adjustment actuators (not shown) to allow at least a portion of the deck 208 to be independently moved relative to the intermediate frame 206. In some embodiments, the deck adjustment actuators may be linear actuators, similar to the height adjustment linear actuators described above, thereby allowing a patient to be supported in various positions. The illustrative patient bed 200 additionally includes siderails 218 that may include one or more interface units 220, which are connected to the controller 104. The interface units 220 may be used by a user (e.g., patient, nurse, etc.) of the patient bed 200 in order to make a number of adjustments to various components driven by electrically coupled components of the patient bed 200. It should be appreciated that additional or alternative user interface units may be provided elsewhere on the patient bed 200, or connected to the patient bed 200 (e.g., a remote control, pendant, etc.).

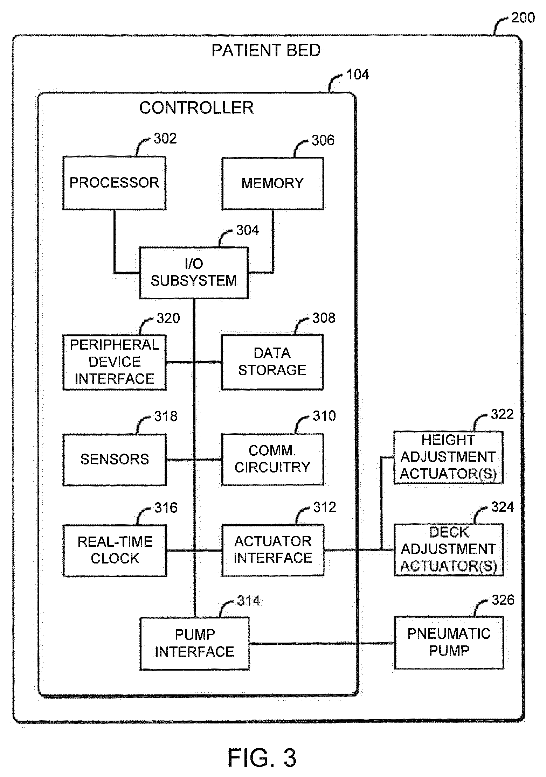

With reference to Fig. 3, a block diagram of an illustrative controller 104 of the patient bed 200 is shown coupled to a number of non-limiting features of the patient bed 200. Of course, in other embodiments, the patient bed 200 may include other or additional functions, such as those commonly found in a patient bed and, as such, the patient bed 200 may include additional or alternative components and/or interfaces for such other or additional functions. As described above, the controller 104 is configured to control the functions (i.e., features) of the patient bed 200. In some embodiments, the information associated with the control signals issued from and/or the commands received by the controller 104 may be stored local to the patient bed 200 and/or transmitted to a remote computing device, such as the patient support management system 110 of Fig. 1, for example.

The illustrative controller 104 includes a processor 302, an input/output (I/O) subsystem 304, a memory 306, a data storage device 308, communication circuitry 310, an actuator interface 312, a pump interface 314, a real-time clock 316, one or more sensors 318, and a peripheral device interface 320. Additionally, in some embodiments, one or more of the illustrative components may be incorporated in, or otherwise form a portion of, another component. For example, the memory 306, or portions thereof, may be incorporated in one or more processors 302 in some embodiments.

The processor 302 may be embodied as any type of processor capable of performing the functions described herein. The processor 302 may be embodied as a single or multi-core processor(s), digital signal processor, microcontroller, or other processor or processing/controlling circuit. The memory 306 may be embodied as any type of volatile or non-volatile memory or data storage capable of performing the functions described herein. In operation, the memory 306 may store various data and software used during operation of the patient bed 200 such as operating systems, applications, programs, libraries, and drivers. The memory 306 is communicatively coupled to the processor 302 via the I/O subsystem 304, which may be embodied as circuitry and/or components to facilitate input/output operations with the processor 302, the memory 306, and other components of the patient bed 200. For example, the I/O subsystem 304 may be embodied as, or otherwise include, memory controller hubs, input/output control hubs, integrated sensor hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 304 may form a portion of a system-on-a-chip (SoC) and be incorporated, along with the processor 302, the memory 306, and other components of the patient bed 200, on a single integrated circuit chip.

The data storage device 308 may be embodied as any type of device or devices configured for short-term or long-term storage of data such as, for example, memory devices and circuits, memory cards, hard disk drives, solid-state drives, or other data storage devices. In some embodiments, the data storage device 308 may be used to store the contents of the patient support information database 106.

The communication circuitry 310 may be embodied as any type of communication circuit, device, or collection thereof, capable of enabling communications between the patient bed 200 and the patient support management system over the network 116. The communication circuitry 310 may be configured to use any one or more communication technologies (e.g., wired or wireless communications) and associated protocols (e.g., Ethernet, Bluetooth®, Wi-Fi®, WiMAX, etc.) to affect such communication.

The actuator interface 312 may be embodied as any type of circuit, device, or collection thereof, capable of communicating with various actuators of the patient bed 200, such as the height adjustment actuators 322 and the deck adjustment actuators 324 of the patient bed 200 as described above. In some embodiments, the actuator interface 312 may provide a serial interface, such as an RS232 interface, controller area network (CAN) interface, or a serial peripheral interface (SPI), for example, for communicating signals between the actuator interface 312 and the patient bed 200.

The pump interface 314 may be embodied as any type of circuit, device, or collection thereof, capable of communicating with various pumps and valves of the patient bed 200, such as the pneumatic pump 326 for controlling the pressure of the bladders of the patient support surface 214 through one or more pressure actuator valves as described above. In some embodiments, the pump interface 314 may provide a serial interface, such as an RS232 interface, controller area network (CAN) interface, or a serial peripheral interface (SPI), for example, for communicating signals between the pump interface 314 and the patient bed 200.

The real-time clock 316 may be embodied as any type of circuit, oscillator, or collection thereof, capable of keeping track of the present time. In some embodiments, the real-time clock 316 may be coupled to an alternative power source to continue to keep time in the event the primary power source for the controller 104 is off or otherwise unavailable.

The one or more sensors 318 may be embodied as any type of sensor capable of sensing or detecting. For example, in some embodiments, the patient bed 200 may include an integrated scale (not shown) in the deck 208, which is comprised of various sensors to detect the presence of a patient and/or a weight of the patient.

The peripheral device interface 320 may be embodied as any type of circuit, device, or collection thereof, capable of interfacing with a peripheral device. For example, the peripheral device interface 320 may provide an interface to a number of subsystems of the patient bed 200 known in the art for monitoring a patient condition, monitoring an operating condition of the patient bed 200, controlling an operating condition of the patient bed 200, and/or providing therapy to patient supported on the patient bed 200. In some embodiments, for example, the peripheral device interface 320 may include a scale system interface, side rail position monitoring system interface, a brake mechanism monitoring system interface, a bed position monitoring system interface, a patient position monitoring system interface including bed exit detection capability, or a therapy device interface, such as a therapeutic mattress. Additionally or alternatively, the peripheral device interface 320 may include various ports, such as universal serial bus (USB) ports, for example, for connecting various peripheral I/O devices (e.g., a display, a touch screen, graphics circuitry, a keyboard, a mouse, a speaker system, etc.) to the controller 104.

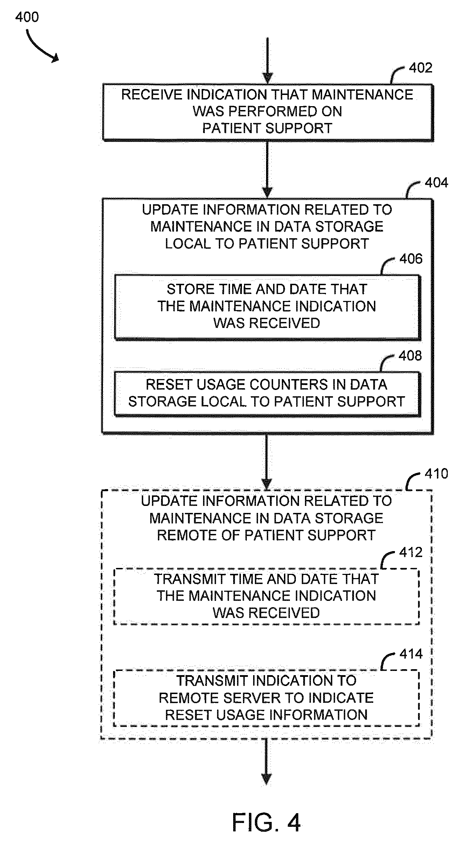

Referring now to Fig. 4, a process 400 for resetting maintenance and usage information is shown that may be performed at a controller 104 of a patient support 102, such as the controller 104 of Fig. 3. Process steps shown in phantom indicate that the particular process step is optional as will be discussed in further detail below. The process 400 begins at step 402, in which an indication is received at the controller 104 that indicates maintenance was performed on a patient support 102. The process 400 proceeds to step 404, wherein the controller 104 stores maintenance related information local to the patient support 102, such as in the patient support information database 106. At step 406 of the process 400, a maintenance date (i.e., present time and date) that corresponds to the time and date at which the indication was received at step 402 is recorded and stored local to the patient support 102 by the controller 104. In some embodiments, the present date may be determined from the real-time clock 316. Alternatively, the present date may be input by a user interface of the patient support 102, or by a peripheral device, such as a handheld computing device carried by the technician that performed the maintenance.

In some embodiments, the maintenance date may be stored for any and/or all of the feature components. In other words, in some embodiments, a single maintenance date may be used, while in other embodiments, more than one maintenance date may be used. For example, an embodiment using the single maintenance date may include a maintenance date that corresponds to a date that maintenance was performed on any one or more components of the patient support 102. Additionally or alternatively, an embodiment using more than one maintenance date may include more than one maintenance dates, wherein each maintenance date corresponds to a date that maintenance was performed for each respective component of the patient support 102.

At step 408, the controller 104 resets usage information counters that correspond to a usage of certain feature components of the patient support 102 in order to represent a usage amount since the maintenance date. In some embodiments, the usage amount may include an actuator usage that corresponds to a number of adjustments of an actuator. For example, the actuator usage may include a number hi/low cycles (i.e., a number of times the lift mechanism 210 moved up/down) of the height adjustment actuators 322 of the lift mechanism 210, an amount of time the lift mechanism 210 has spent in operation, and/or a number of adjustments of the deck adjustment actuators 324 of the deck 208. Additionally or alternatively, in some embodiments, the usage amount may include usage of pressure components (e.g., the pneumatic pump 326), such as a pneumatic pump usage and/or a pressure actuator valve usage, for example.

In some embodiments, the process 400 proceeds to step 410, wherein the controller 104 stores maintenance related information of the patient support 102 remotely, such as in the patient support maintenance database 112 of the patient support management system 110. To do so, at step 412, the maintenance date of step 406 may be transmitted to the patient support management system 110 to update a corresponding maintenance date of the patient support 102 stored at the patient support management system 110. Additionally, at step 414, the controller 104 transmits an indication (i.e., an availability status) to the patient support management system 110 to indicate to the patient support management system 110 to reset any usage information corresponding to the patient support 102.

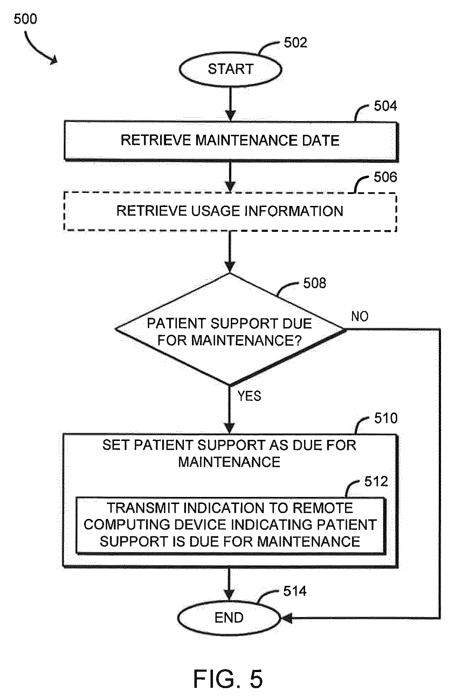

Referring now to Fig. 5, a process 500 for determining whether a patient support 102 is due for maintenance is shown that may be performed at a controller 104 of a patient support 102, such as the controller 104 of Fig. 3. The process is initialized at step 502, which may be initiated by a timer of the patient support 102 (i.e., a polling technique), an action performed at the patient support 102 (i.e., an event-driven technique), and/or by a request received from a remote computing device, such as the patient support management system 110 of Fig. 1.

The process 500 proceeds to step 504, wherein the controller 104 retrieves the maintenance date. In some embodiments, the maintenance date may be stored in the patient support information database 106. In some embodiments, the process 500 may proceed to step 506, wherein the controller 104 may retrieve usage information. The process 500 then proceeds to decision step 508 to determine whether the patient support 102 is due for maintenance. As described above, in some embodiments, determining whether the patient support 102 is due for maintenance may be determined based on the maintenance date retrieved at step 504 and/or the usage information retrieved at step 506. For example, regularly scheduled, preventative maintenance may be necessary after a predetermined period of time after the most recent regularly scheduled, preventative maintenance was performed. As such, the controller 104 may rely on the maintenance date retrieved at step 504 to determine whether the patient support 102 is due for maintenance. In another example, one or more components may require maintenance after a predetermined number of uses. As such, the controller 104 may rely on the usage information for the one or more components to determine whether the patient support 102 is due for maintenance. Accordingly, in some embodiments, the controller may rely on the maintenance date retrieved at step 504 to determine the regularly scheduled, preventative maintenance, but may pre-empt the regularly scheduled, preventative maintenance based on usage of the patient support 102.

If the patient support 102 is not due for maintenance, the process 500 progresses to step 514, where the process 500 terminates. If the patient support 102 is due for maintenance, the process 500 proceeds to step 510 to set the support as due for maintenance. At step 512, the controller 104 transmits an indication (i.e., an availability status) to a remote computing device, such as the patient support management system 110, which indicates that the patient support 102 is due for maintenance. From step 512, the process 500 proceeds to step 514, where the process 500 terminates.

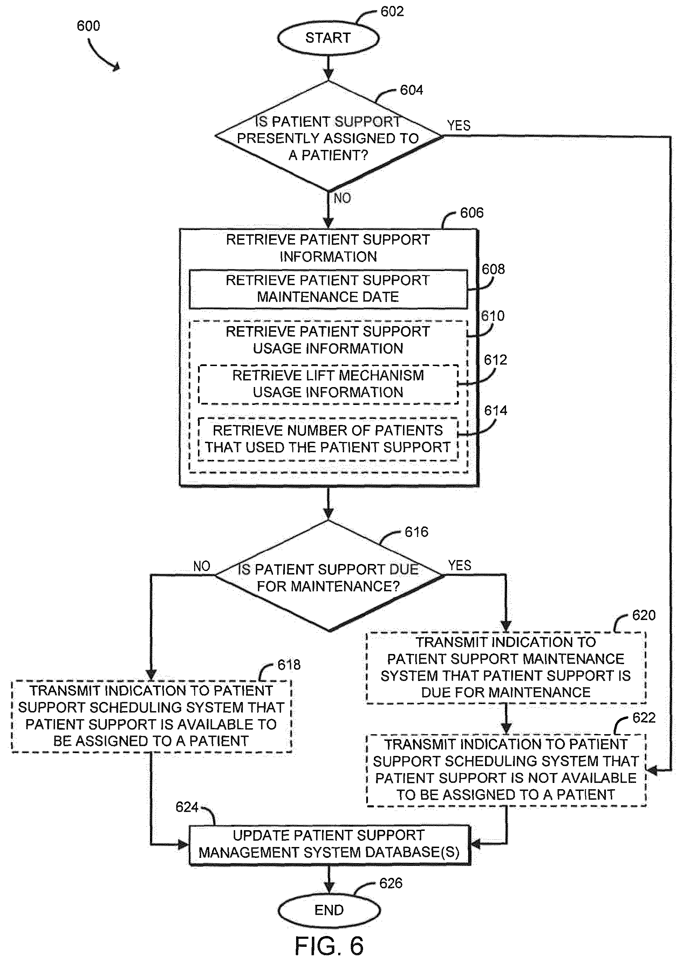

Referring now to Fig. 6, a process 600 for determining whether a patient support 102 is due for maintenance is shown that may be performed at a remote computing device, such as the patient support management system 110 of Fig. 1. The process is initialized at step 602, which may be initiated by a timer of the patient support 102 designated to check the state of the patient support 102 at predetermined time intervals (i.e., polling) and/or a received request (i.e., event-driven) from the patient support 102, the patient support maintenance system 120, or the patient support scheduling system 130.

The process 600 proceeds to step 604 to determine whether a patient support 102 is presently assigned to a patient. In some embodiments, the patient support management system 110 may query the status of the patient support 102 from the patient support scheduling database 114 to determine whether the patient support 102 is presently assigned to a patient. If the patient support 102 is presently assigned to a patient, the process 600 progresses to step 622, wherein the patient support management system 110 transmits an indication (i.e., an availability status) to the patient support scheduling system 130 that the patient support 102 is not available to be assigned to a patient. If the patient support 102 is not presently assigned to a patient, the process 600 proceeds to step 606, wherein the patient support management system 110 retrieves information corresponding to the patient support 102. At block 608, the patient support management system 110 retrieves the maintenance date corresponding to the patient support 102. In some embodiments, the patient support management system 110 may additionally or alternatively retrieve patient support 102 usage information corresponding to the patient support 102. For example, in some embodiments, at block 612 the patient support management system 110 may retrieve lift mechanism 210 usage information, such as hi/low cycle information, lift mechanism 210 run-time, and/or a number of lift mechanism 210 activations since the maintenance date. It should be appreciated that, while the lift mechanism 210 usage information has been retrieved by the patient support management system 110 at block 612, the patient support management system 110 may retrieve any usage information that may be used to determine when maintenance is due. For example, such usage information may include various switch activations, motor run-times, valve actuations, compressor run times, side rail movements, brake actuations, motorized drive wheel run time, cardiopulmonary resuscitation (CPR) handle usage, and/or other run times, cycles, or activations of patient support 102 components. In another example, in some embodiments, at block 614 the patient support management system 110 may retrieve a number of patients that used the patient support 102 since the maintenance date.

The process 600 proceeds to step 616, wherein the patient support management system 110 determines whether the patient support 102 is due for maintenance based on the patient support 102 information retrieved at step 606. As described above, the determination of whether the patient support 102 is due for maintenance may be based on the maintenance date retrieved at step 608 and/or the usage information retrieved at step 610. In some embodiments, the patient support management system 110 may determine whether a predetermined duration has passed since the most recent maintenance date received from the patient support 102 to determine whether the patient support 102 is due for maintenance. Additionally or alternatively, in some embodiments, the patient support management system 110 may determine whether a usage threshold has been passed to determine whether the patient support 102 is due for maintenance, regardless of whether the predetermined duration has passed since the most recent maintenance date. For example, in an embodiment that tracks the number of hi/low cycles since the maintenance date, the patient support management system 110 may determine the number of hi/low cycles has exceeded a hi/low cycle threshold and, as such, have determined the patient support 102 should be scheduled for maintenance, despite not having exceeded the predetermined duration.

In some embodiments, the patient support maintenance system 120 and/or the patient support scheduling system 130 may additionally or alternatively include localized databases that may be periodically synchronized with the patient support maintenance database 112 and/or the patient support scheduling database 114. As such, in some embodiments, if the patient support management system 110 determines the patient support 102 is not due for maintenance, the process 600 may proceed to step 618, wherein the patient support management system 110 may transmit an indication to the patient support scheduling system 130 that indicates the patient support 102 is available to be assigned to a patient. If the patient support management system 110 determines the patient support 102 is due for maintenance, the process 600 may proceed to step 620, wherein the patient support management system 110 may transmit an indication to the patient support maintenance system 120 that indicates the patient support 102 is due for maintenance. From step 620, process 600 may proceed to step 622, wherein the patient support management system 110 may additionally or alternatively transmit an indication to the patient support scheduling system 130 that indicates the patient support 102 is not available to be assigned to a patient.

After transmitting the indications at either step 618 or step 622, the process 600 proceeds to step 624, wherein the patient support management system 110 updates the patient support maintenance database 112 and/or the patient support scheduling database 114 as necessary to update the present assignment availability of patient support 102. For example, the patient support maintenance database 112 may include a table that provides a listing of each patient support 102 that is presently due for maintenance, while the patient support scheduling database 114 may include a table that provides a listing of each patient support 102 that is presently available to be assigned to a patient. The process 600 then proceeds to step 626, wherein the process 600 terminates.

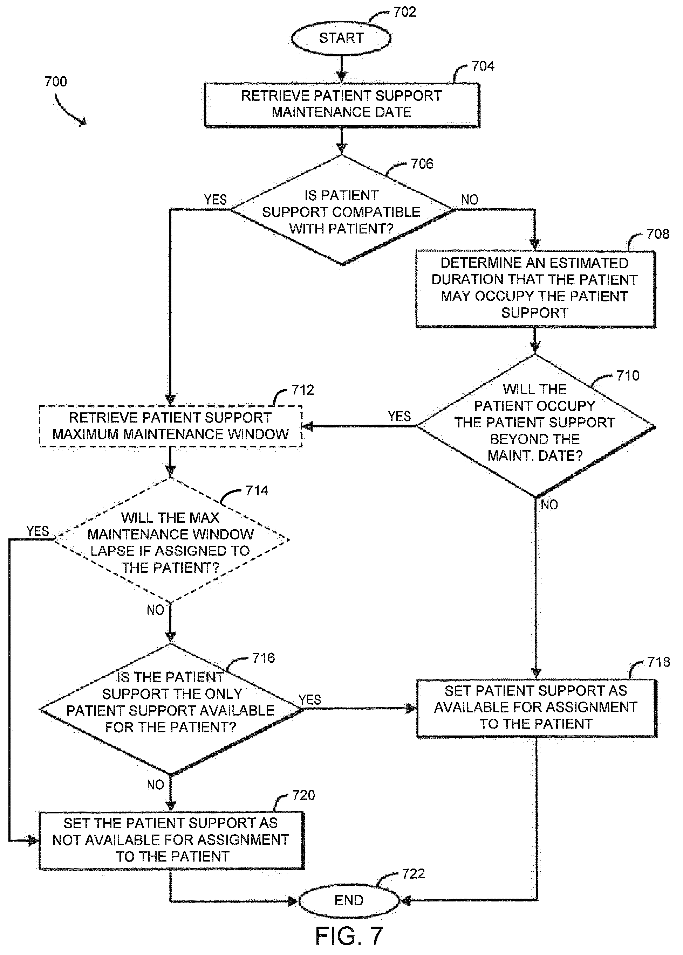

Referring now to Fig. 7, a process 700 for determining whether a patient support 102 is available for being scheduled to a patient is shown that may be performed at a remote computing device, such as the patient support scheduling system 130 of Fig. 1. The process is initialized at step 702, which may be initiated by scheduling software running on one or more of the scheduling computing devices 132, for example. For example, as described previously, the patient support scheduling system 130 may be embodied as an ADT system. In such an embodiment, the process 700 may be initialized upon receiving a request for patient assignment at the patient support scheduling system 130 upon the patient being processed for admission. The process 700 proceeds to step 704, wherein the patient support scheduling system 130 retrieves the maintenance data that corresponds to the patient support 102. In some embodiments, the patient support scheduling system 130 may query the patient support maintenance database 112 to retrieve the maintenance data.

The process 700 proceeds to decision step 706, wherein the patient support scheduling system 130 determines whether the patient support 102 is compatible with the patient based on one or more capabilities of the patient support 102 and attributes of the patient in order to assign the patient support 102 to a patient that has capabilities suitable for the healthcare attributes of the patient. If the patient support scheduling system 130 determines that the patient support 102 is not compatible with the patient, the process 700 proceeds to step 708, wherein the patient support scheduling system 130 determines an estimated duration that the patient may occupy the patient support 102. The process 700 then proceeds to decision block 710, wherein the patient support scheduling system 130 determines whether the patient support 102 will be occupied beyond the maintenance date. In other words, the patient support scheduling system 130 determines whether assigning the patient to the patient support 102 may cause the patient support 102 to be occupied when the patient support 102 would become due for maintenance.

If the patient support scheduling system 130 determines that the patient support 102 is compatible with the patient, in some embodiments, the process 700 may proceed to step 712, wherein the patient support scheduling system 130 my retrieve a maximum maintenance window for the patient support 102. In other words, in some embodiments, a threshold duration (i.e., the maximum maintenance window) may indicate a required period of time in which maintenance on the patient support 102 may be performed, and that may not be exceeded. In such an embodiment, keeping the patient in the patient support 102 may risk malfunction, which may be to the detriment of the patient. As such, the process 700 may proceed to step 714, wherein the patient support scheduling system 130 determines whether the maximum maintenance window might lapse if the patient support 102 is assigned to the patient. If not, the process 700 proceeds to decision step 716, wherein the patient support scheduling system 130 determines whether the patient support 102 is the only patient support available for the patient. Otherwise, the process 700 proceeds to step 720, wherein the patient support scheduling system 130 sets the patient support 102 as not available for assignment to the patient.

At step 716, if the patient support scheduling system 130 determines the patient support 102 is the only patient support 102 available for the patient, the process 700 proceeds to step 718. At step 718, the patient support scheduling system 130 sets the patient support 102 as available for assignment to the patient before proceeding to step 722, wherein the process 700 terminates. If the patient support scheduling system 130 determines the patient support 102 is not the only patient support 102 available for the patient, the process 700 proceeds to step 720 wherein the patient support 103 is set as not available for assignment to the patient before the process 700 proceeds to step 722, wherein the process 700 terminates.

Although certain illustrative embodiments have been described in detail above, variations and modifications exist.

Embodiments of the invention can be described with reference to the following numbered clauses, with preferred features laid out in the dependent clauses:
1. A patient support apparatus comprising: a frame that includes a base frame coupled to an intermediate frame configured to move vertically relative to the base frame, and a deck coupled to the intermediate frame configured to move between a plurality of positions relative to the intermediate frame; a controller in electrical communication with one or more components to control movement of the intermediate frame and the deck, wherein the controller includes: an interface to receive an indication that maintenance was performed on the patient support apparatus; a data storage device to store a maintenance date that corresponds to a date and time the indication was received; and communication circuitry operable to transmit the maintenance date via a network to a remote computing device to determine an availability status of the patient support apparatus to indicate whether the patient support apparatus is available to be scheduled for maintenance or assigned to a patient, wherein the availability status is based on the maintenance date.
2. The patient support apparatus of clause 1, wherein the one or more components of the patient support apparatus include at least one of a valve, an actuator, and a sensor.
3. The patient support apparatus of clause 1, wherein the data storage device is further to store a usage data of the patient support apparatus, and wherein the communication circuitry is further operable to transmit the usage data to the remote computing device via the network to determine the availability status of the patient support apparatus based on the usage data.
4. The patient support apparatus of clause 3, further comprising: an inflatable mattress supported by the deck; a pump to inflate the inflatable mattress; and a pressure actuator valve to control a pressure of the inflatable mattress, the pressure actuator valve being fluidly coupled between the inflatable mattress and the pump, wherein the controller is in electrical communication with the pressure actuator valve to provide a signal to cause the pressure actuator valve to adjust the pressure of the inflatable mattress, wherein the usage data includes a pressure actuator valve usage based at least in part on a number of times the pressure actuator valve was used since the maintenance date.
5. The patient support apparatus of clause 3, further comprising: a lift mechanism that includes one or more lift arms that interconnect the base frame and the intermediate frame to cause the intermediate frame to move relative to the base frame, wherein the controller is in electrical communication with the one or more lift arms to provide signals to the one or more lift arms to control the movement of the intermediate frame relative to the base frame, and wherein the usage data includes an actuator usage of the lift mechanism.
6. The patient support apparatus of clause 5, wherein the actuator usage of the lift mechanism includes at least one of a number of hi/low cycles that indicate a number of times the lift mechanism moved the intermediate frame from hi to low positions since the maintenance date.
7. The patient support apparatus of clause 5, wherein the actuator usage of the lift mechanism includes at least one of a number of hi/low cycles that indicate an amount of time the lift mechanism has spent in operation since the maintenance date.
8. The patient support apparatus of clause 5, wherein the actuator usage of the lift mechanism includes at least one of a number of hi/low cycles that indicate a number of activations of the lift mechanism since the maintenance date.
9. The patient support apparatus of clause 3, further comprising: one or more actuators to control the movement of the deck between a plurality of positions relative to the intermediate frame, wherein the controller is in electrical communication with the one or more actuators to provide signals to the one or more actuators to control the movement of the deck between the plurality of positions, and wherein the usage data includes an actuator usage amount that indicate a number of times an actuator has been used to move a respective deck since the maintenance date.
10. The patient support apparatus of clause 9, wherein the actuator usage includes at least one of a number of complete cycles that indicate a number of times the actuator has moved the deck between a first position and a second position since the maintenance date.
11. The patient support apparatus of clause 9, wherein the actuator usage includes an amount of time the actuator has spent in operation since the maintenance date since the maintenance date.
12. The patient support apparatus of clause 5, wherein the actuator usage includes a number of activations of the actuator since the maintenance date.
13. The patient support apparatus of clause 1, wherein the data storage device is further to store a usage data of the patient support apparatus that corresponds to a usage of the one or more components of the patient support apparatus, and wherein the communication circuitry is further operable to transmit the usage data to the remote computing device via the network to determine the availability status of the patient support apparatus based on the maintenance date and the usage data.
14. The patient support apparatus of clause 1, wherein the controller further includes a real-time clock, and wherein the controller is further to determine whether the patient support apparatus is due for maintenance and to transmit an indication to the remote computing device that indicates the patient support apparatus should be scheduled for maintenance in response to a determination that the patient support apparatus is due for maintenance.
15. The patient support apparatus of clause 14, wherein the determination that the patient support apparatus is due for maintenance comprises a determination that a predetermined amount of time has elapsed since the maintenance date based on a present value of the real-time clock.
16. A system for managing availability of a patient support for scheduling the patient support, the system comprising: a patient support that includes a frame including one or more components controlled by a controller to move portions of the frame between a plurality of positions; a remote computing device to manage whether the patient support is to be set as scheduled for maintenance or set available for assignment to a patient; wherein the controller includes an interface to receive an indication that maintenance was performed on the patient support apparatus, a data storage device to store a maintenance date that corresponds to a date and time the indication was received, and communication circuitry operable to communicate with the remote computing device to transmit the maintenance date to the remote computing device, and wherein the remote computing device is to determine whether the patient support apparatus is available to be scheduled for maintenance or assigned to a patient based on the maintenance date.
17. The system of clause 16, wherein the remote computing device is further to provide an availability status that indicates to schedule the patient support apparatus for maintenance in response to a determination that the patient support apparatus is not presently assigned to a patient and is due for maintenance.
18. The system of clause 16, wherein the remote computing device is further to provide an availability status that indicates the patient support apparatus is available to be assigned to a patient in response to a determination that the patient support apparatus is presently assigned to a patient or is not due for maintenance.
19. The system of clause 16, wherein the controller further includes a real-time clock and the controller is further configured to determine whether the patient support is due for maintenance based on a comparison of a present time determined from the real-time clock and the maintenance date, and wherein the patient support is further to transmit an indication to the remote computing device that indicates the patient support apparatus should be scheduled for maintenance in response to a determination that an amount of time has passed since the maintenance date that exceeds a predetermined duration.
20. The system of clause 16, wherein the data storage device of the controller is further configured to store usage information that corresponds to a usage of the one or more components of the frame, and wherein the patient support is further to transmit the usage information to the remote computing device.
21. The system of clause 20, wherein the remote computing device is further to provide an availability status that indicates to schedule the patient support apparatus for maintenance in response to a determination that the patient support apparatus is not presently assigned to a patient and the usage information indicates a usage threshold was exceeded.
22. The system of clause 20, wherein the remote computing device is further to provide an availability status that indicates the patient support apparatus is available to be assigned to a patient in response to a determination that the patient support apparatus is presently assigned to a patient or the usage information indicates a usage threshold was not exceeded.

## Claims

1. A patient support apparatus comprising:
a frame that includes a base frame coupled to an intermediate frame configured to move vertically relative to the base frame, and a deck coupled to the intermediate frame configured to move between a plurality of positions relative to the intermediate frame;
a controller in electrical communication with one or more controllable components including at least one of a valve, an actuator, and a sensor, wherein the controller includes:
a data storage device to store a usage data of the patient support apparatus, wherein the controller provides signals to cause operation of the one or more controllable components, wherein the usage data includes usage of the one or more controllable components, and wherein the controller further includes:
communication circuitry operable to transmit the usage data to a remote computing device via the network to determine the availability status of the patient support apparatus based on the usage data.

2. The patient support apparatus of claim 1, further comprising: an inflatable mattress supported by the deck, and a pump to inflate the inflatable mattress, wherein the one or more controllable components include a pressure actuator valve to control a pressure of the inflatable mattress, the pressure actuator valve being fluidly coupled between the inflatable mattress and the pump, wherein the controller is in electrical communication with the pressure actuator valve to provide a signal to cause the pressure actuator valve to adjust the pressure of the inflatable mattress, and wherein the usage data includes a pressure actuator valve.

3. The patient support apparatus of either claim 1 or claim 2, further comprising a lift mechanism that includes one or more lift arms that interconnect the base frame and the intermediate frame to cause the intermediate frame to move relative to the base frame, wherein the one or more controllable components include the one or more lift arms, wherein the controller is in electrical communication with the one or more lift arms to provide signals to the one or more lift arms to control the movement of the intermediate frame relative to the base frame, and wherein the usage data includes an actuator usage of the lift mechanism.

4. The patient support apparatus of claim 3, wherein the actuator usage of the lift mechanism includes at least one of a number of hi/low cycles that indicate a number of times the lift mechanism moved the intermediate frame from hi to low positions.

5. The patient support apparatus of claim 3, wherein the actuator usage of the lift mechanism includes at least one of a number of hi/low cycles that indicate an amount of time the lift mechanism has spent in operation.

6. The patient support apparatus of claim 3, wherein the actuator usage of the lift mechanism includes at least one of a number of hi/low cycles that indicate a number of activations of the lift mechanism.

7. The patient support apparatus of any preceding claim, wherein the one or more controllable components include one or more actuators to control the movement of the deck between a plurality of positions relative to the intermediate frame, wherein the controller is in electrical communication with the one or more actuators to provide signals to the one or more actuators to control the movement of the deck between the plurality of positions, and wherein the usage data includes an actuator usage amount that indicate a number of times an actuator has been used to move a respective deck.

8. The patient support apparatus of claim 7, wherein the actuator usage includes at least one of a number of complete cycles that indicate a number of times the actuator has moved the deck between a first position and a second position.

9. The patient support apparatus of claim 7, wherein the actuator usage includes an amount of time the actuator has spent in operation since the maintenance date.

10. The patient support apparatus of claim 7, wherein the actuator usage includes a number of activations of the actuator.

11. The patient support apparatus of any preceding claim, wherein the remote computing device is further operable to provide an availability status that indicates the patient support apparatus is available to be assigned to a patient in response to a determination that the patient support apparatus is presently assigned to a patient or the usage data indicates a usage threshold was not exceeded.

12. The patient support apparatus of any preceding claim, including an interface to receive an indication that maintenance was performed on the patient support apparatus, wherein the data storage device is further to store a maintenance date that corresponds to a date and time the indication was received and wherein the communication circuitry is further operable to transmit the maintenance date via a network to the remote computing device to determine an availability status of the patient support apparatus to indicate whether the patient support apparatus is available to be scheduled for maintenance or assigned to a patient, wherein the availability status is based on the maintenance date.

13. The patient support apparatus of claim 12, wherein the communication circuitry is further operable to transmit the usage data to the remote computing device via the network to determine the availability status of the patient support apparatus based on the maintenance date and the usage data.

14. The patient support apparatus of either claim 12 or claim 13 wherein the controllable component usage is since the maintenance date.

15. The patient support apparatus of any one of claims 12 to 14, wherein the controller further includes a real-time clock, and wherein the controller is further to determine whether the patient support apparatus is due for maintenance and to transmit an indication to the remote computing device that indicates the patient support apparatus should be scheduled for maintenance in response to a determination that the patient support apparatus is due for maintenance.
